# EUROPEAN PATENT APPLICATION

(11) **EP 2 615 459 A1**
(43) Date of publication of application: **17.07.2013**
(21) Application number: 12166013.8
(22) Date of filing: 27.04.2012
(51) Int. Cl.: G01N 33/68

(54) **proSP-B based diagnosis of distal airway involvement in asthma**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention relates to the field of diagnostic measures. Specifically, it relates to a method for diagnosing whether a subject suffering from asthma exhibits distal bronchoalveolar damage, said method comprises determining the amount of the biomarker proSP-B in a sample of said subject, and comparing said amount to a reference whereby distal bronchoalveolar damage is to be diagnosed. Further encompassed are diagnostic devices and kits for carrying out the aforementioned method.

## Description

The present invention relates to the field of diagnostic measures. Specifically, it relates to a method for diagnosing whether a subject suffering from asthma exhibits distal bronchoalveolar damage, said method comprises determining the amount of the biomarker proSP-B in a sample of said subject, and comparing said amount to a reference whereby distal bronchoalveolar damage is to be diagnosed. Further encompassed are diagnostic devices and kits for carrying out the aforementioned method.

Asthma bronchiale is a disease of the lung believed to be present in approximately 7 % of the population in developed countries. Clinically asthma is characterized by coughing, shortness of breath, chest tightness and wheezing.

Asthma is associated with airway inflammation of the mucosa, submucosa and adventitia associated with cellular infiltration, particularly, of eosinophils and activated helper T cells (Fanta 2009, NEJM: 360: 1002 - 1014).

Treatment of asthma includes, for example, quick acting beta adrenergic drugs, inhaled or systemic steroids, long acting beta agonist bronchodilatators, leucotriene modifiers and anti-IgE therapies, which have led to stepwise algorithm in the treatment of asthma (Fanta loc cit.).

Asthma is, however, a heterogenous disease and not all patients respond well to a therapy. In this context targeting the distal parts of the lung is a specific challenge, which can be best accomplished by using hydrofluoroalkane solutions of inhalated drugs with spacers and valved holding chambers (Dolovich 1999, Can Respir J 6: 290 - 295).

Asthma is believed to predominantly be a disease of large, central airways. However, there is conclusive evidence that the disease also involves the lung periphery. When peripheral airways are affected, airways become narrow and airway closure may occur. Such airway closure is frequently caused by fibrin production associated with enhanced fibrin deposition which may ultimately alter surfactant function However, airway closure of the distal airways can not be recognizes by standard imaging techniques such as chest X-ray imaging (Kaminsky 2011, Pulmonary Pharmacology and Therapeutics 24: 199 - 202).

As outlined above, there is a need to identify peripheral involvement of asthma in order to identify patients suffering therefrom and to choose an appropriate therapy for these patients. Several therapeutic approaches are known for asthma patients (see, e.g., Lazarus 2010, NEJM 363(8): 755764; Fahy 2010, NEJM 363(23): 2233-2247; Corren 2011, NEJM 365(12): 1088-1098).

Surfactant Proteins and, in particular, SP-B have been reported to be biomarkers for lung diseases or disorders. Increased expression of SP-A and SP-B has been reported, e.g., in budesonide treated asthma mice (Zu 2008, Allergy Asthma Proc. 29(5): 486-492). In lavage samples of children suffering from pulmonary alveolar proteinosis or chronic respirator distress, proSP-B was found to be increased (Griese 2005, Respiratory Research 6: 80). WO1999/133337 discloses that SP-B can be used for the diagnosis of the extent of lung damage. A correlation of SP-B levels in lavage of asthma patients after allergen challenge has been also reported (Erpenbeck 2006, Allergy 61(5): 598-604).

The technical problem underlying the present invention can be seen as the provision of means and methods for reliably and efficiently diagnosing distal bronchoaleveolar damage in subjects suffering from asthma. The technical problem is solved by the embodiments characterized in the claims and herein below.

Thus, the present invention relates to a method for diagnosing whether a subject suffering from asthma exhibits distal bronchoalveolar damage comprising:
a) determining the amount of the biomarker proSP-B in a sample of said subject; and
b) comparing said amount to a reference whereby distal bronchoalveolar damage is to be diagnosed.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein means assessing whether a subject suffers from one or more medical conditions, preferably from distal bronchoalveolar damage. The term "diagnosing" as used herein also includes making a determination, according to the systems and methods disclosed herein, that a subject does not suffer from one or more medical conditions, such as distal bronchoalveolar damage. As will be understood by those skilled in the art, such an assessment is usually not intended to be correct for 100% of the subjects to be diagnosed. The term, however, requires that assessment of the presence or absence of a distal bronchoalveolar damage is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "distal bronchoalveolar damage" refers to a damage or injury of the bronchoalveolar barrier present between the airways and the blood vessels at the distal parts of the bronchoalveolar airways. Said distal parts of the bronchoalveolar airways include the alveoli. In said case, the barrier is established by the so-called alveolar-capillary membrane. However, the term also encompasses the small airways, such as the bronchioles and, in particular, the distal parts of the bronchioles. Said distal parts of the airways, preferably, have a diameter of about 2 mm or less. Damage or injury as referred to herein encompass any alteration of the permeability of the broncheoalveolar barrier compared to the physiological (i.e. non-pathological) state. The damage, preferably, results in an increase in the amount of surfactant proteins which are normally present in the surfactant of the airways in the blood. The damage or injury of the distal bronchoalveloar airways as referred to before may result, preferably, from fibrin depositions.

The term "asthma" or "asthma bronchiale" is a disease of the airways of the lung. The disease is associated with airway inflammation of the mucosa, submucosa and adventitia. In particular, the inflammation is characterized by cellular infiltration of eosinophils and activated T helper cells. Clinical symptoms of asthma involve, e.g., coughing, dyspnea, chest tightness, and wheezing. The disease, usually, involves the large, central airways. However, in some subjects, there is also involvement of the more distal airways in the lung periphery. The asthma in the lung periphery, however, also involves severe airway narrowing and/or closure as well as fibrin deposition. Subjects which suffer from asthma with mere involvement of the distal airways in the lung periphery can not be easily identified by standard imaging techniques such as chest X-ray imaging.

The term "subject" as used herein relates to animals, preferably mammals, and, more preferably, humans. The subject according to the present invention will, in general, suffer from asthma as described elsewhere herein. Preferably, said subject does not exhibit one or more other diseases, disorders or life style behaviours selected from the group consisting of: cardiac complications, preferably, severe chronic artery disease, preferably patients with symptoms of NYHA class II onwards, and backward heart failure, lung diseases other than asthma bronchiale, preferably, chronic obstructive pulmonary disease (COPD), pneumonia, current smokers, current intoxications by substances, preferably, by drugs, such as bleomycin or metho-threxat, lung cancer, acute systemic infections and kidney disease. Backward heart failure as used herein means the ability of the heart to pump blood at a sufficient rate only when heart filling pressures are abnormally high, i.e. the problem is primarily increased venous back pressure behind the heart afterload. The concept of backward heart failure contends that in heart failure, the right or left ventricle fails to discharge its contents or fails to fill normally. As a consequence, the pressures in the atrium and venous system behind the failing ventricle rise, and retention of sodium and water occurs as a consequence of the elevation of systemic venous and capillary pressures and the resultant transudation of fluids into the interstitial space.

It is known that several diseases, disorders or life style behaviours, such as mentioned above, can also cause an elevation of the biomarker proSP-B in the blood. Accordingly, the method of the present invention shall, preferably, not be applied to those subjects. If the clinical history of a subject to be investigated by the method of the present invention with respect to the aforementioned diseases, disorders or life style behaviours is unknown, the subject may, in a preferred embodiment of the method of the present invention, be tested for the presence of the said disease, disorders and/or life style behaviours as set forth elsewhere herein

The term "sample" refers to a sample of a body fluid. Samples of body fluids can be obtained by well known techniques and include, preferably, samples of blood, plasma, serum, buccal cells, or urine, more preferably, samples of blood, plasma or serum.

The term "proSP-B" relates to a precursor polypeptide of the Surfactant Protein B (SP-B). SP-B is synthesised as a precursor, i.e. proSP-B, and the mature SP-B is obtained by proteolytic cleavage. The mature SP-B is a 79-amino acid hydrophobic peptide that facilitates the stability and rapid spreading of surfactant phospholipids during respiratory cycles. It maintains the molecular continuity of the monolayer of the lipid and peptide at the air-water interface during breathing and facilitates the incorporation of lipids from the lung aqueous subphase into the lipid monolayer at the alveolar air-water interface. Since mature SP-B is comprised in the proSP-B in its N-terminal portion, proSP-B is, preferably, determined by detecting the presence or absence of the C-terminal portion of the proSP-B polypeptide, i.e. C-terminal proSP-B. C-terminal proSP-B in the sense of the present invention relates to proSP-B and all cleavage products or fragments thereof comprising the C-terminal sequence of full length proSP-B. Accordingly, C-terminal proSP-B includes but is not limited to the following: (i) full length proSP-B, i.e. proSP-B comprising the N-terminal propeptide including the amino acid sequence of mature SP-B and the C-terminal proSP-B, (ii) the mid-molecular portion and the C-terminal fragment, i.e. the amino acids from position 201 to 381of proSP-B and (iii) the C-terminal proSP-B fragment, i.e. the amino acids from position 280 to 381 of proSP-B. Amino acid sequences comprising amino acids 1 to 381 for proSP-B are disclosed in Johansson 1992, FEBS Lett. 301:165-167, Jacobs 1987, J Biol Chem 262(20): 9808-11 and Jacobs 1988, J Biol Chem 263(2): 1093, or Pilot-Matias 1989, DNA 8:75-86 and are deposited in Uni-ProtKB/Swiss-Prot data base under accession numbers P07988; Q96R04 or Genbank accession number P07988.3. proSP-B as used herein encompasses also variants of the aforementioned specific proSP-B polypeptides. Such variants have at least the same essential biological and immunological properties as the specific proSP-B polypeptides. In particular, they share the same essential biological and immunological properties if they are detectable by the same specific assays referred to in this specification, e.g., by ELISA assays using polyclonal or monoclonal antibodies specifically recognizing the said proSP-B polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific proSP-B polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The comparison window, preferably, is the entire length of the query sequence or at least 50% of its length. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific proSP-B polypeptides or the aforementioned types of variants as long as these fragments have the essential immunological and biological properties as referred to above. Such fragments may be, e.g., degradation products of the proSP-B polypeptides. Further included are variants which differ due to posttranslational modifications such as phosphorylation or myristylation. Moreover, the aforementioned proSP-B may be present as a monomer and/or in dimerized form.

According to some embodiments, the amino acid sequence identity will typically be highest in critical regions of the marker which (in general) account for the biological activity, or are involved in the determination of three-dimensional configuration, ultimately responsible for the biological activity of the marker. As such, it should be understood that certain amino acids substitutions, deletions, or additions are acceptable and can be expected, for example, if these substitutions (deletions or additions) are in regions which are not critical to activity or do not affect the three-dimensional configuration of the marker molecule. For example, in some embodiments of the instant disclosure, the amino acid sequence of the marker may comprise a conservative amino acid substitution. Amino acids may be placed in the following classes: non-polar, uncharged polar, basic, and acidic. A form of conservative substitution includes an amino acid of one class being replaced with another amino acid of the same type (such that the substitution does not materially alter the biological activity and/or three-dimensional configuration of the marker molecule). Table 1 provides an exemplary listing of examples of amino acids belonging to each class.

**Table 1. Exemplary listing of examples of amino acids belonging to each class.**

| **Class of Amino Acid** | **Examples of Amino Acids** |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |

Determining the amount of proSP-B or any other peptide or polypeptide referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR-analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) preferably, removing non-bound ligand and other components which may be present in the sample, (c) measuring the amount of bound ligand, i.e. the complex of the peptide and the ligand formed in step (a). The bound ligand, i.e. the ligand or the ligand/peptide complex, will generate an intensity signal which reflects the amount of peptide or polypeptide originally present in the sample. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to, i.e. cross-react with, another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative.

Binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemiluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other detection methods as described above.

Further suitable techniques for the determination of a polypeptide or peptide are described in the following.

The amount of a peptide or polypeptide may be, also preferably, determined as follows: (a) contacting a solid support comprising a ligand for the peptide or polypeptide as specified above with a sample comprising the peptide or polypeptide, (b) preferably, removing unbound peptide or polypeptide as well as remaining sample material and (c) measuring the amount peptide or polypeptide which is bound to the support. Preferably, the amount of the complex of the ligand and the peptide or polypeptide formed on the solid support is measured. It will be understood that the amount of the complex formed during the determination shall represent the amount of the peptide or polypeptide originally present in the sample. The ligand is, preferably chosen from the group consisting of nucleic acids, peptides, polypeptides, antibodies and aptamers and is, preferably, present on a solid support in immobilized form. As with other embodiments of the disclosure discussed herein, the step of measuring the amount of peptide bound to the support may be accomplished through the use of a second ligand specific for the peptide or polypeptide (which remains present following step b), for example, as bound to the ligand bound with the solid support). The second ligand may specifically recognize a different recognition epitope of the peptide or polypeptide than the ligand bound with the solid support. Further, the second ligand may also include one or more "tags" as already described and disclosed herein. Also, as with other embodiments of the disclosure discussed herein, a secondary ligand which specifically recognizes and binds to either the ligand (e.g., as described with respect to step a)) or the second ligand (as described above) may be utilized for the measuring step. Also as noted herein, such secondary ligand may include one or more of the tags described herein. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analysed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a subject suffering from asthma has distal bronchoalveolar damage. Therefore, the reference amount is to be chosen so that either a difference or a similarity in the compared amounts allows identifying those test subjects which belong into the group of subjects suffering from asthma and having distal broncheoalveolar damage, or not.

Accordingly, the term "reference amount" as used herein refers to an amount which allows assessing whether a subject suffering from acute inflammation has an increased risk for mortality. Accordingly, the reference may, e.g., be derived from (i) a subject or group of subjects suffering from asthma known to exhibit distal bronchoalveolar damage, (ii) a subject or group of subjects suffering from asthma known to not exhibit distal bronchoalveolar damage or (iii) a a clinically apparently healthy subject or a group of such subjects. The reference amount may be used to define and establish a threshold amount. The threshold amount, preferably, allows for a rule-in and/or a rule-out diagnosis. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analysed together, i.e. simultaneously or subsequently, with the test sample. It should also be understood that, the system and methods disclosed herein may utilize more than one of "reference amount" as selected from the exemplary groups provided above. For example, a system or method according to the instant disclosure may utilize a reference amount derived from a healthy group of subjects and a reference amount from a group of subjects suffering from asthma known to exhibit distal bronchoalveolar damage.

In one embodiment of the method of the present invention, said reference is derived from a subject or group of subjects suffering from asthma known to exhibit distal bronchoalveolar damage or is derived from a subject or group of subjects which is apparently clinically healthy. In such a case, preferably, an essentially identical or decreased amount for the biomarker in the sample compared to the reference is indicative for a subject which does not exhibit distal bronchoalveolar damage and wherein an increased amount for the biomarker in the sample compared to the reference is indicative for a subject which exhibits distal bronchoalveolar damage.

In yet another embodiment of the method of the present invention, said reference is derived from a subject or a group of subjects suffering from asthma known to exhibit distal broncheoalveolar damage. Preferably, an essentially identical or increased amount for the biomarker in the sample compared to the reference is indicative for a subject which exhibits distal bronchoalveolar damage and wherein a decreased amount for the biomarker in the sample compared to the reference is indicative for a subject which does not exhibit distal bronchoalveolar damage.

Reference amounts can be calculated for a cohort of subjects (i.e. (i) subjects suffering from asthma known to exhibit distal bronchoalveolar or (ii) subjects suffering from asthma known to not exhibit distal bronchoalveolar or (iii) clinically apparently healthy subjects) based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom.

Preferably, the median values for a biomarker determined in a reference population as specified above may be also used as a basis for establishing thresholds. More preferably, the median of about 43 ng/ml was found in accordance with the present invention for proSP-B in a clinically apparently healthy population of subjects. Also preferably, the values for the 25^{th} or 75^{th} percentile for proSP-B found in this clinically apparently healthy population, i.e. about 42 ng/ml or about 45 ng/ml, may be used as well. More preferably, the median of about 68 ng/ml was found in accordance with the present invention for C fragment proSP-B in a clinically apparently healthy population of subjects.

"About" as used in accordance with the present invention means +/- 20%, +/- 10%, +/- 5%, +-2 % or +-/ 1% from the said value.

The method of the present invention may also include a step of establishing a diagnosis whether or not a subject exhibits distal bronchoalveolar damage based on the result of the comparison described above.

In principle, the present invention also relates to a method of diagnosing whether whether a subject suffering from asthma exhibits distal bronchoalveolar damage comprising diagnosing said distal bronchoalveolar damage in a subject based on a comparison to a reference of the amount of the biomarker proSP-B determined in a sample of said subject

Advantageously, it has been found in the studies underlying the present invention that proSP-B in a body fluid such as blood, plasma or serum can serve as a biomarker that allows for identifying distal broncheoalveolar damage in subjects suffering from asthma. Thanks to the present invention, it is possible to identify those patients with asthma and peripheral lung involvement. The conventional diagnostic approaches based on X-ray imaging systems cannot resolve the peripheral obstructions and are, therefore, not suitable for establishing a diagnosis of asthma with peripheral lung involvement. The reliable and efficient diagnosis of distal bronchoalveolar damage and, hence, the peripheral lung involvement, allows for an individual therapeutic intervention. Specifically, asthma with peripheral lung involvement usually requires therapeutic measures being different from those used for conventional asthma with involvement of the central, large airways. For example, inhalation-based drug therapies may be used only the inhaled drugs contain hydroxyfluoroalkane solutions together with spacers and/or valved holding chambers. Furthermore, systemic therapies using, e.g., steroids, are most often more effective in such patients.

In an aspect of the invention, a method for establishing an aid for diagnosing whether a subject suffering from asthma exhibits distal bronchoalveolar damage, or not, is contemplated, said method comprising:
a) determining the amount of the biomarker proSP-B in a sample of said subject, said determining comprises (i) bringing the sample into contact with a detection agent that specifically binds to proSP-B for a time sufficient to allow for the formation of a complex of the said detection agent and the proSP-B from the sample, (ii) measuring the amount of the formed complex, wherein the said amount of the formed complex is proportional to the amount of proSP-B present in the sample, and (iii) transforming the amount of the formed complex into an amount of proSP-B reflecting the amount of proSP-B present in the sample;
b) comparing said amount to a reference; and
c) establishing an aid for diagnosing distal bronchoalveolar damage based on the result of the comparison made in step b).

A suitable detection agent may be, in an aspect, an antibody which is specifically binds to proSP-B in a sample of a subject to be investigated by the method of the invention. Another detection agent that can be applied, in an aspect, may be an aptamere which specifically binds to proSP-B in the sample. In yet an aspect the, sample is removed from the complex formed between the detection agent and the proSP-B prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the proSP-B present in the sample. It will be understood that the specificity and/or sensitivity of the detection agent to be applied defines the degree of proportion of proSP-B comprised in the sample which is capable of being specifically bound. Further details on how the determination can be carried out are also found elsewhere herein. The amount of formed complex shall be transformed into an amount of proSP-B reflecting the amount indeed present in the sample. Such an amount, in an aspect, may be essentially the amount present in the sample or may be, in another aspect, an amount which is a certain proportion thereof due to the relationship between the formed complex and the amount present in the original sample. In yet an aspect of the aforementioned method, step a) may be carried out by an analyzing unit, in an aspect, an analyzing unit as defined elsewhere herein. In other aspects, any or all of steps a) through c) may be carried out by an analyzing unit as defined elsewhere herein. In an aspect of the method of the invention, the amount determined in step a) is compared to a reference. In an aspect, the reference is a reference as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used is reflected properly. In an aspect, the comparison is carried out automatically, e.g., assisted by a computer system or the like.

The aid for diagnosing is established based on the comparison carried out in step b) by allocating the subject either into a group of subjects suffering distal bronchoalveolar damage with certain likelihood or a group of subjects not suffering therefrom. As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. Thus, the method may establish an aid of diagnosis which may, in an aspect, require further strengthening of the diagnosis by other techniques. In an aspect of the invention, the aid for diagnosing is established automatically, e.g., assisted by a computer system or the like.

In an aspect of the method of the invention, said method further comprises a step of treating / prescribing / recommending or managing the subject according to the result of the aid of diagnosis established in step c). Such a recommendation may, in an aspect, be an adaptation of life style, nutrition and the like aiming to improve the life circumstances, the application of therapeutic measures as set forth elsewhere herein in detail, and/or a regular disease monitoring.

In an aspect of the aforementioned method, steps b) and/or c) are carried out by an evaluation unit as set forth elsewhere herein.

It is to be understood that the definitions and explanations of the terms made above and below apply accordingly for all embodiments described in this specification and the accompanying claims.

In a preferred embodiment of the aforementioned method, the amount of a natriuretic peptide and/or a cardiac troponin is determined in addition to proSP-B.

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms described elsewhere herein. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chim-ica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac troponin" refers to all troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac troponin refers to troponin T and/or troponin I, and, most preferably, to troponin T. It is to be understood that isoforms of troponins may be determined in the method of the present invention together, i.e. simultaneously or sequentially, or individually, i.e. without determining the other isoform at all. Amino acid sequences for human troponin T and human troponin I are disclosed in Anderson, loc cit and Ferrieres 1998, Clinical Chemistry, 44: 487-493. The term "cardiac troponin" encompasses also variants of the aforementioned specific troponins, i.e., preferably, of tropoinin T or troponin I. Such variants have at least the same essential biological and immunological properties as the specific cardiac troponins. In particular, they share the same essential biological and immunological properties if they are detectable by the same specific assays referred to in this specification, e.g., by ELISA Assays using polyclonal or monoclonal antibodies specifically recognizing the said cardiac troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific troponin. The degree of identity between two amino acid sequences, in principle, can be determined by algorithms described elsewhere herein. Variants may be allelic variants or any other species specific homologs, paralogs, or orthologs. Moreover, the variants referred to herein include fragments of the specific cardiac troponins or the aforementioned types of variants as long as these fragments have the essential immunological and biological properties as referred to above. Such fragments may be, e.g., degradation products of the troponins. Further included are variants which differ due to posttranslational modifications such as phosphorylation or myristylation.

By determining the amount of a natriuretic peptide and/ore a cardiac troponin, the presence or absence of a cardiac complication can be diagnosed. As discussed already elsewhere herein, the proSP-B level in the blood may be influenced by cardiac complications. Accordingly, the additional determination of the cardiac biomarkers referred to above (i.e. the natriuretic peptides and cardiac troponins) allows for making the diagnosis more reliable since an increase in the blood levels of proSP-B can be allocated to the distal bronchoalveolar damage that accompanies asthma rather than the result of a cardiac complication. Thus, an increase in the blood levels of the aforementioned cardiac biomarkers could be indicative for a cardiac complication, such as heart failure.

In order to determine increased levels for the cardiac biomarkers, a reference amount can be used which is derived from at least one of, or both of (i) subjects suffering from a cardiac complication or (ii) subjects suffering known to not suffer from a cardiac complication, and, preferably clinically apparently healthy subjects.

An essentially identical or increased amount for the biomarkers in the sample compared to the reference derived from subjects suffering from a cardiac complication is indicative for a subject exhibiting or exhibiting in addition to asthma a cardiac complication while a decreased amount for the biomarkers in the sample compared to the reference is indicative for a subject exhibiting no cardiac complication.

Alternatively, the said reference is derived from subjects suffering known to not suffer from a cardiac complication, and, preferably clinically apparently healthy subjects. An essentially identical or decreased amount for the biomarkers in a sample compared to the reference is indicative for a subject exhibiting no cardiac complication while an increased amount for the biomarkers in the sample compared to the reference is indicative for a subject exhibiting or exhibiting in addition to asthma a cardiac complication.

Suitable references for the aforementioned biomarkers which may serve as thresholds can be calculated as described for proSP-B, above. The considerations made in this context apply mutatis mutandis for the biomarkers NT-proBNP and cardiac troponin. The threshold can by establishing a ROC for the cohort of subjects as described above and deriving a threshold amount therefrom.

Surprisingly, it has been found in accordance with the present invention that in patients exhibiting mild cardiac disease such as patients with coronary artery disease exhibiting symptoms up to NYHA class II did not show altered proSP-B levels due to the cardiac disease. A thershold value for NT-proBNP which preferably can be used for identifying patients having also a cardiac disease which influences blood proSP-B levels and which can be used in the method according to the invention is about 227 pg/ml. Accordingly, if an amount for NT-proBNP equal or less than 227 pg/ml is determined, there shall be no significant contribution to the proSP-B by the cardiac disease. Thus, the diagnosis made according to the method of the present invention is further strengthened.

In another preferred embodiment of the method of the present invention, said method further comprises treating (or recommending/ prescribing) the patient suffering from distal bronchoalveolar asthma with a specific therapy, if the subject is diagnosed with distal bronchoalveolar damage.

The term "distal bronchoalveolar asthma therapy" as used herein refers to therapeutic measures which aim to cure or ameliorate distal bronchoalveolar damage, asthma or symptoms accompanied therewith. Preferably, said distal broncheoaleveolar asthma therapy is selected from the group consisting of: Systemic administration of steroids, administration of inhalation drugs comprising hydroxyfluoroalkane solutions with spacers and/or valved holding chambers, systemic antileucotrien administration, systemic administration cromones, systemic anti-IgE (Omalizumab) administration, systemic CCR3 administration, systemic administration of anti-interleukine 13 (IL-13), and systemic administration of anti-cholinergs, preferably, muscarin receptor agonists or theophylline.

Moreover, in another preferred embodiment of the method of the present invention, said method further comprises treating with / prescribing / recommending an inhalation asthma therapy, if it is diagnosed that the subject does not exhibit distal bronchoalveolar damage.

An "inhalation asthma therapy" as referred to herein is a therapy which involves the inhalation of a drug in order to cure or ameliorate asthma or the symptoms accompanying it. Preferably, said inhalation asthma therapy is selected from the group consisting of: bronchodilatators and beta agonists, preferably albuterol, terbuterol, short or long acting nebulizers.

In general, the present invention contemplates the use se of the biomarker proSP-B or a detection agent that specifically binds thereto in a sample of a subject suffering from asthma for diagnosing whether said subject exhibits distal bronchoalveolar damage. Moreover, included is also the use of a combination of proSP-B and NT-proBNP and/or a cardiac troponin or a combination of detection agents therefor for diagnosing whether said subject exhibits distal bronchoalveolar damage.

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

The present invention relates to a device adapted for carrying out the method of the present invention comprising
a) an analyzing unit comprising a detection agent which specifically binds to proSP-B adapted for determining the amount of proSP-B in a sample of a subject suffering from asthma; and
b) an evaluation unit for comparing the determined amount with a reference whereby it can be diagnosed whether a subject exhibits distal broncheoaleveolar damage, said unit comprising a database with reference values as defined in herein above and a computer-implemented algorithm for carrying out a comparison as defined herein above.

The term "device" as used herein relates to a system comprising the aforementioned units operatively linked to each other as to allow the diagnosis or monitoring according to the methods of the invention. Preferred detection agents which can be used for the analysing unit are disclosed elsewhere herein. The analysing unit, preferably, comprises said detection agents in immobilized form on a solid support which is to be contacted to the sample comprising the biomarkers the amount of which is to be determined. Moreover, the analysing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references are either derived from a subj ect or group of subj ects as defined above in context with the method of the present invention. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

In a preferred embodiment of the device of the present invention, said analysing unit further comprises a detection agent which specifically binds NT pro-BNP and/or a cardiac troponin in a sample of a subject suffering from asthma.

According to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

The present invention also relates to a kit adapted for carrying out the method of the present invention comprising a detection agent for the biomarker proSP-B, at least one standard for a reference and instructions for carrying out the said method. Additionally, kits, including some or all of the components necessary to practicing one or more of the methods disclosed herein, are provided. A kit, according to the instant disclosure, may be made of any suitable material. Non-limiting examples of kit materials are card-board or other paper product, plastic, glass, wood, metal, and any alloy thereof.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a pre-defined amount for proSP-B polypeptide representing a reference amount. Such a standard may represent, e.g., the amount of proSP-B from a subject or group of subjects suffering from asthma known to exhibit distal bronchoalveolar or a subject or group of subjects suffering from asthma known to not exhibit distal bronchoalveolar or a clinically apparently healthy subject or group thereof.

In a preferred embodiment of the the kit of the invention, said kit further comprises a detection agent for the biomarker NT-proBNP and/or a cardiac troponin.
In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s0 is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

All references cited in this specification are herewith incorporated by reference with respect to their entire disclosure content and the disclosure content specifically mentioned in this specification.

### FIGURES

**Figure 1** shows the correlation between proSP-B and the C-fragment of proSP-B established by a regression analysis for asthma bronchiale patients.
**Figure 2** shows the correlation between proSP-B and the arterial partial oxygen pressure in the blood (p02) of asthma bronchiale patients. A tendency of higher proSP-B levels to lower p02 is apparent in the regression analysis which is consistent with involvement of peripheral airways and the alveoli.

### EXAMPLES

The invention shall be now illustrated by Examples. These Examples, whatsoever, must not be construed as to limit the scope of the invention.

### Example 1: Determination of biomarkers proSP-B, C-fragment of proSP-B, NT-proBNP and sensitive troponin T (hsTnT) in patients with asthma bronchiale and clinically healthy controls

A total of 35 patients with asthma bronchiale were included into the study at the time of acute exacerbation of asthma and shortness of breath. None of the patients had clinical evidence of pneumnia and had also no history of kidney disease, acute exacerbation were treated first with rochodilatators and in case of nonresponse with systemic steroids (100 mg prednisolone i.v.). The median age of the patients with asthma was 42 years.

Sixty nine apparently clinically healthy subjects served as a control group.

A total of 79 patients with chronic artery disease (CAD) who did not smoke und did not have a lung or kidney disorder or an acute infection and who displayed symptoms that were at most compatible to NYHA II (at its worst) were included as a further cardiac control group.

In blood serum samples of the asthma patients, the cardiac control group, and the control group, proSP-B, NT-proBNP and Troponin T was determined.

ProSP-B was tested using newly developed proSP-B tests aiming at the detection of proSP-B and the terminal C fragment of proSP-B (C fragment proSPB).

The proSP-B assay uses a mouse monoclonal anti-proSP-B (N-terminus) antibody as a capture and a mouse monoclonal anti-proSP-B (C-terminus) antibody as a detection reagent. The assay principle is a sandwich format. The antibody to the N-terminal pro-sequence binds to an epitope comprised in the peptide sequence ranging from amino acid 160 to 169 of proSP-B. The antibody to the C-terminal pro-sequence binds to an epitope comprised in the peptide sequence ranging from amino acid 323 to 334 of proSP-B. Detection is based on an electrochemiluminescence immunoassay (ECLIA), using a Tris(bipyridyl)-ruthenium(II) complex as label. For carrying out the assay, the biotinylated capture antibody (80 µl), the ruthenium-labeled detection antibody (80 µl), and sample or standard material (10 µl) are incubated in homogeneous phase for 9 min at 37°C. Concentrations in the stock solution were 1.7 µg/ml for the biotinylated capture antibody and 1.2 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for proSP-B is given in pg/ml.

The C-fragment proSP-B assay uses a first mouse monoclonal anti-proSP-B (C-terminus) antibody as a capture and a second mouse monoclonal anti proSP-B (C-terminus) antibody as a detection reagent. The assay principle is a sandwich format. The antibody to the first C-terminal pro-sequence binds to an epitope comprised in the peptide sequence ranging from amino acid 323 to 334 of proSP-B. The antibody to the second C-terminal pro-sequence binds to an epitope comprised in the peptide sequence ranging from amino acid 285 to 294 of proSP-B. Detection is based on an electrochemiluminescence immunoassay (ECLIA), using a Tris(bipyridyl)-ruthenium(II) complex as label. For carrying out the assay, the biotinylated capture antibody (MAB 1.7.41) (80 µl), the ruthenium-labeled detection antibody (MAB 1.3.9) (80 µl), and sample or standard material (10 µl) are incubated in homogeneous phase for 9 min at 37°C. Concentrations in the stock solution were 1.5 µg/ml for the biotinylated capture antibody and 1.0 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for C-terminal proSP-B is given in pg/ml.

NT-pro BNP and sensitive Troponin T were determined using commercially available ELECSYS tests.

NT-proBNP was determined with sandwich immunoassays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. NT-proBNP amounts between 2 pg/ml and 35,000 pg/ml can be measured.

Troponin T (hsTNT) was also tested using Roche analysers, the test follows the same test principles as described for NT-pro BNP. The high sensitivity Troponin T test used in this study has a sensitivity of 2 pg/ml and can be used on ELECSYS 2010 as well as on Cobas e411 an cobas e601 analysers.

### Example 2: proSP-B and C fragment proSP-B have similar diagnostic potentials

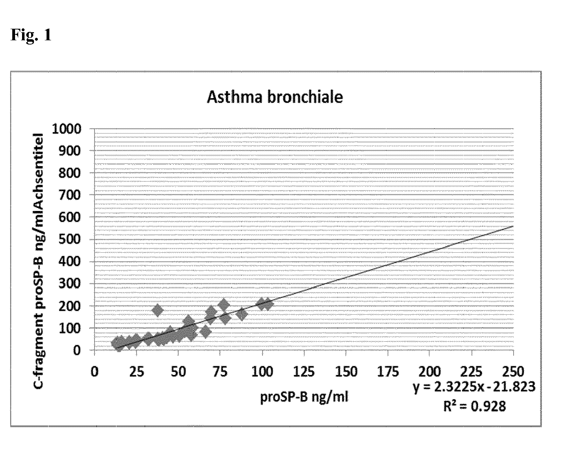

The diagnostic potential of the new proSP-B tests for proSP-B and C fragment proSP-B was compared by analysing the measured amounts by a regression analysis. Both tests gave comparable results. As shown in Fig. 1 proSP-B and C fragment proSP-B identified the same patients and, thus, revealed identical diagnostic information.

### Example 3: proSP-B is a biomarker for peripheral lung involvement in asthma bronchiale patients

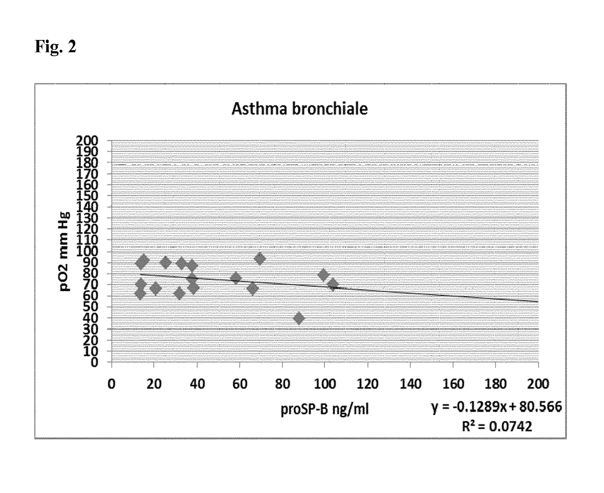

Fig. 2 shows that when arterial p02 was correlated to pro SP-B, there was a trend of higher proSP-B levels to lower p02. This result is consistent with involvement of peripheral airways and the alveoli in asthma.

The following Table shows the individual results obtained for the investigated patients:

| | **years** | **ng/ml** | **ng/ml** | **pg/ml** | **pg/ml** | **Diagnosis therapy** | |
|---|---|---|---|---|---|---|---|
| **Pat.Id.** | **Age** | **proSP-B** | **C-fragment prosP-B** | **proBNP** | **hsTnT** | * | ** |
| **I-01/1** | **45** | **58.25** | **99.90** | **146.20** | **0.10** | **pa** | |
| **I-15/1** | **30** | **57.87** | **67.64** | **120.52** | **0.00** | **pa** | |
| **I-27/1** | **53** | **77.25** | **203.13** | **121.14** | **0.00** | **c** | **+** |
| **I-32/1** | **55** | **24.55** | **44.87** | **31.97** | **2.20** | **c** | |
| **I-33/1** | **26** | **50.93** | **63.32** | **13.00** | **0.00** | **pa** | |
| **I-36/1** | **54** | **56.19** | **128.60** | **34.35** | **0.00** | **pa** | |
| **I-41/1** | **41** | **47.06** | **59.33** | **5.00** | **0.00** | **pa** | |
| **I-44/1** | **63** | **54.17** | **86.40** | **74.82** | **6.56** | **c** | |
| **I-46/1** | **28** | **32.66** | **48.73** | **5.00** | **0.00** | **a** | |
| **I-56/1** | **43** | **40.72** | **54.48** | **85.95** | **0.00** | **a** | |
| **I-61/1** | **59** | **78.41** | **142.79** | **7.84** | **0.00** | **pa** | |
| **II-008/1** | **38** | **42.96** | **56.84** | **94.20** | **0.00** | **a** | |
| **II-024/1** | **27** | **31.86** | **50.88** | **67.79** | **0.00** | **a** | |
| **II-027/1** | **26** | **99.55** | **205.25** | **5.00** | **0.00** | **pa** | **+** |
| **II-028/1** | **42** | **20.58** | **31.75** | **5.00** | **0.00** | **a** | |
| **II-029/1** | **32** | **37.82** | **50.46** | **37.70** | **0.00** | **a** | |
| **II-031/1** | **48** | **20.90** | **37.43** | **17.78** | **0.00** | **a** | |
| **II-032/1** | **75** | **103.67** | **205.11** | **37.58** | **0.00** | **pa** | **+** |
| **II-033/1** | **50** | **66.45** | **83.14** | **14.44** | **0.00** | **pa** | |
| **II-040/1** | **37** | **87.92** | **159.95** | **94.56** | **0.00** | **pa** | |
| **II-042/1** | **28** | **38.42** | **46.36** | **5.00** | **0.00** | **a** | |
| **II-044/1** | **50** | **45.19** | **78.70** | **195.88** | **0.00** | **pa** | |
| **II-065/1** | **42** | **25.54** | **41.38** | **29.07** | **0.00** | **a** | |
| **II-076/1** | **76** | **69.53** | **143.01** | **135.45** | **13.86** | **c** | **+** |
| **II-077/1** | **29** | **15.05** | **22.92** | **50.79** | **0.00** | **a** | |
| **II-086/1** | **29** | **264.93** | **620.43** | **41.26** | **0.00** | **pa** | **+** |
| **II-087/1** | **29** | **41.52** | **49.30** | **26.94** | **0.00** | **a** | |
| **II-129/1** | **59** | **69.80** | **170.55** | **84.65** | **4.40** | **c** | |
| **II-146/1** | **25** | **13.82** | **21.17** | **5.00** | **0.00** | **a** | |
| **II-156/1** | **30** | **15.36** | **22.05** | **5.00** | **0.00** | **a** | |
| **II-169/1** | **47** | **13.58** | **33.89** | **5.00** | **0.00** | **a** | |
| **II-196/1** | **44** | **14.02** | **26.56** | **13.65** | **0.00** | **a** | |
| **II-217/1** | **25** | **24.58** | **35.40** | **24.10** | **0.00** | **a** | |
| **II-221/1** | **64** | **37.77** | **178.10** | **5.00** | **0.00** | **a** | |
| **II-233/1** | **47** | **16.21** | **35.44** | **24.39** | **3.00** | **c** | |

| | | | | | | | |
|---|---|---|---|---|---|---|---|
| *= diagnosis based on biomarker; **= systemic therapy responders; pa=asthma with distal bronchoalveolar damage or injury; a= asthma without distal bronchoalveolar damage or injury; c= ambiguous because of possible more severe cardiac complications; +=responder to systemic steroid therapy. | | | | | | | |

As can be seen from the Table the majority of patients were rather young and had NT-pro BNP values within the normal range and undetectable sensitive Troponin T and, thus, had no evidence of severer abnormal cardiac function. Moreover, patients with asthma had similar levels of these biomarkers when compared to the apparently healthy control group.

In the apparently healthy control group, the median for proSP-B was 43 ng/ml and the 25^{th} percentile was 42 ng/ml, the 75^{th} percentile 45 ng/ml. The median for C fragment proSP-B was somewhat higher at 68 ng/ml. The median for proSP-B has been used as reference value discriminating the asthma patients in those with and those without peripheral lung involvement. The established diagnoses are indicated in the table above. Moreover, peripheral lung involvement was confirmed for at least some of the patients by their response to systemic steroid therapy (100 mg prednisolone i.v.).

In the cardiac control group (patients with CAD displaying up to NYHA II symptoms), no elevation for proSP-B and C fragment pro SP-B has been found. The observed medians were 35 ng/ml for proSP-B and 65 ng/ml for C fragment proSP-B, respectively. The median for NT-proBNP in such cardiac control patients showing normal proSP-B levels in the blood was 227 pg/ml. Elevation of proSP-B is to observed, however, in the more severe cardiac patients with symptoms of NYHA class III or more.

The present study shows that proSP-B identifies a subgroup of patients with involvement of peripheral airways and the alveoli which is information that can not be obtained with other methods. Moreover, proSP-B, in this context, identifies the extent (severity) of distal airway involvement since a linear correlation could be established by regression analysis. The majority of patients are young and had no evidence of cardiac diseases which may affect proSP-B levels in the blood.

Further, the cardiac control group shows that no or only insignificant elevation of proSP-B is observable in CAD patients with mild symptoms up to NYHA class II. Onlypatients with more severe cardiac disease such as severe cases of NYHA class II or NYHA class III onwards with significantly elevated NT-pro BNP or sensitive Troponin T could exhibit a cardiac contribution to the observed proSP-B levels in blood. Thus, only if a level for NT-proBNP is detected which is above the threshold for NT-proBNP found in the moderate CAD patients investigated in the present study, it is recommended to also take into account a cardiac contribution to the proSP-B levels in blood. Consequently a high proSP-B level may not necessarily indicate a peripheral lung involvement in such asthma patients.

In summary, proSP-B offers for the first time a test which captures distal broncheoalveolar damage or injuries accompanying asthma which can not be recognized by conventional imaging techniques. The presence of absence of involvement of distal airways or alveoli has therapeutic consequences since inhaled drugs, in general, do not reach the distal airways. Thus, patients with elevated proSP-B (depending on the extent of distal airway involvement) require systemic treatment, e.g. by administering steroids, or inhaled drugs that contain hydrofluoroalkane solutions together with spacers and valved holding chambers or both. In contrast, patients with normal or near normal proSP-B can be treated with inhaled drugs and, thus, benefit from reduced side effects.

### FURTHER EMBODIMENTS

The following illustrative embodiments illustrate various embodiments within the scope of the instant disclosure, but are not intended to be and should not be construed as a complete list of all embodiments within the scope of the present disclosure.
1. A method for diagnosing distal bronchoalveolar damage in a subject, comprising:
   a) contacting, in vitro, a portion of a sample from the subject with a ligand immunoreactive for a proSP-B biomarker;
   b) determining an amount of the proSP-B biomarker in the portion of the sample of said subject;
   c) comparing said amount determined in said step of determining to a proSP-B reference value indicative of no distal bronchoalveolar damage; and
   d) providing a diagnosis of distal bronchoalveolar damage if said amount determined in said step of determining is at least 5 ng/ml greater than the reference value.
2. The method of 1, wherein said reference is derived from a subject or group of subjects known to be free of distal bronchoalveolar damage.
3. The method of 2, wherein an essentially identical or decreased amount for the biomarker in the sample compared to the reference is indicative for a subject which does not exhibit distal bronchoalveolar damage.
4. The method of 1, wherein the ligand is specifically immunoreactive for the proSP-B biomarker C-terminus.
5. The method of 4, wherein said step of providing a diagnosis of distal bronchoalveolar damage comprises providing said diagnosis of distal bronchoalveolar damage if said amount determined in said step of determining is at least 10 ng/ml greater than the reference value.
6. The method of any one of 1 to 5, wherein said steps of contacting and determining are performed by a device adapted for the performing immunoreactive assays and said steps of comparing and providing are performed by a computing device operatively communicative with said device adapted for performing immunoreactive assays.
7. The method of any one of 1 to 3 wherein said method further comprises treating the subject with one of a systemic administration of steroids, administration of inhalation drugs comprising hydroxyfluoroalkane solutions with spacers and/or valved holding chambers, systemic antileucotrien administration, systemic administration cromones, systemic anti-IgE (Omalizumab) administration, systemic CCR3 administration, systemic administration of anti-interleukine 13 (IL-13), and systemic administration of anti-cholinergs, preferably, muscarin receptor agonists and theophylline, if said amount determined in said step of determining is at least 5 ng/ml greater than the reference value.
8. The method of any one of 1 to 3 wherein said method further comprises treating the subject with an inhalation asthma therapy, if said amount determined in said step of determining is one of less than, equal to, or less than 5 ng/ml greater than the reference value.
10. The method of 8, wherein said inhalation asthma therapy is selected from the group consisting of: Bronchodilatators and beta agonists, preferably albuterol, terbuterol, short or long acting nebulizers.
11. The method of any one of 4 and 5 wherein said method further comprises treating the subject with one of a systemic administration of steroids, administration of inhalation drugs comprising hydroxyfluoroalkane solutions with spacers and/or valved holding chambers, systemic antileucotrien administration, systemic administration cromones, systemic anti-IgE (Omalizumab) administration, systemic CCR3 administration, systemic administration of anti-interleukine 13 (IL-13), and systemic administration of anti-cholinergs, preferably, muscarin receptor agonists and theophylline, if said amount determined in said step of determining is at least 10 ng/ml greater than the reference value.
12. The method of any one of 4 and 5 wherein said method further comprises treating the subject with an inhalation asthma therapy, if said amount determined in said step of determining is one of less than, equal to, or less than 10 ng/ml greater than the reference value.
13. The method of 12, wherein said inhalation asthma therapy is selected from the group consisting of: Bronchodilatators and beta agonists, preferably albuterol, terbuterol, short or long acting nebulizers.
14. The method of any of 1 to 5, wherein amount of a natriuretic peptide and/or a cardiac troponin is determined in addition to proSP-B.
15. A method of treating a subject suffering from distal bronchoalveolar damage comprising:
   a) contacting, in vitro, a portion of a sample from the subject with a ligand immunoreactive for a proSP-B biomarker;
   b) determining an amount of the proSP-B biomarker in the portion of the sample of said subject;
   c) comparing said amount determined in said step of determining to a proSP-B reference value indicative of no distal bronchoalveolar damage; and
   d) treating the subject with one of a systemic administration of steroids, administration of inhalation drugs comprising hydroxyfluoroalkane solutions with spacers and/or valved holding chambers, systemic antileucotrien administration, systemic administration cromones, systemic anti-IgE (Omalizumab) administration, systemic CCR3 administration, systemic administration of anti-interleukine 13 (IL-13), and systemic administration of anti-cholinergs, preferably, muscarin receptor agonists and theophylline when the comparing step indicates said amount determined in said step of determining is at least 5 ng/ml greater than the reference value.
16. The method of 15, wherein the ligand is specifically immunoreactive for the proSP-B biomarker C-terminus.
17. The method of 16, wherein said step of treating comprises treating the subject if said amount determined in said step of determining is at least 10 ng/ml greater than the reference value.
18. A system adapted for facilitating a therapeutic decision in a subject, comprising:
   means for contacting, in vitro, a portion of a sample from the subject with a first ligand immunoreactive for a proSP-B biomarker;
   means for determining an amount of the proSP-B biomarker in the portion of the sample of said subject;
   a computing device having a processor; and
   a non-transient machine readable media including a plurality of instructions executable by the processor, the instructions when executed compare the amount of proSP-B biomarker in the portion of the sample to a proSP-B reference value indicative of no distal bronchoalveolar damage and provide an output representing a need for a therapy for distal bronchoalveolar damage in the subject if the amount of the proSP-B biomarker in the portion of the sample is at least 5 ng/ml greater than the reference value.
19. The system of 18, wherein the ligand is specifically immunoreactive for the proSP-B biomarker C-terminus.
20. The system of 19, wherein the instructions provide an output representing a need for a therapy for distal bronchoalveolar damage in the subject if the amount of the proSP-B biomarker in the portion of the sampleis at least 10 ng/ml greater than the reference value.

## Claims

1. A method for diagnosing whether a subject suffering from asthma exhibits distal bronchoalveolar damage comprising:
a) determining the amount of the biomarker proSP-B in a sample of said subject; and
b) comparing said amount to a reference whereby distal bronchoalveolar damage is to be diagnosed.

2. The method of claim 1, wherein said reference is derived from a subject or group of subjects suffering from asthma known to exhibit distal bronchoalveolar damage or is derived from a subject or group of subjects which is apparently clinically healthy.

3. The method of claim 2, wherein an essentially identical or decreased amount for the biomarker in the sample compared to the reference is indicative for a subject which does not exhibit distal bronchoalveolar damage and wherein an increased amount for the biomarker in the sample compared to the reference is indicative for a subject which exhibits distal bronchoalveolar damage.

4. The method of claim 1, wherein said reference is derived from a subject or a group of subjects suffering from asthma known to exhibit distal bronchoalveolar damage.

5. The method of claim 4, wherein an essentially identical or increased amount for the biomarker in the sample compared to the reference is indicative for a subject which exhibits distal bronchoalveolar damage and wherein a decreased amount for the biomarker in the sample compared to the reference is indicative for a subject which does not exhibit distal bronchoalveolar damage.

6. The method of any one of claims 1 to 5, wherein the amount of a natriuretic peptide and/or a cardiac troponin is determined in addition to proSP-B.

7. The method of any one of claims 1 to 6 wherein said method further comprises recommending a distal bronchoalveolar asthma therapy, if it is diagnosed that the subject exhibits distal bronchoalveolar damage.

8. The method of claim 7, wherein said distal bronchoaleeolar asthma therapy is selected from the group consisting of: Systemic administration of steroids, administration of inhalation drugs comprising hydroxyfluoroalkane solutions with spacers and/or valved holding chambers, systemic antileucotrien administration, systemic administration cromones, systemic anti-IgE (Omalizumab) administration, systemic CCR3 administration, systemic administration of anti-interleukine 13 (IL-13), and systemic administration of anti-cholinergs, preferably, muscarin receptor agonists or theophylline.

9. The method of any one of claims 1 to 6 wherein said method further comprises recommending an inhalation asthma therapy, if it is diagnosed that the subject does not exhibit distal bronchoalveolar damage.

10. The method of claim 8, wherein said inhalation asthma therapy is selected from the group consisting of: Bronchodilatators and beta agonists, preferably albuterol, terbuterol, short or long acting nebulizers.

11. Use of the biomarker proSP-B or a detection agent that specifically binds thereto in a sample of a subject suffering from asthma for diagnosing whether said subject exhibits distal bronchoalveolar damage.

12. A device adapted for carrying out the method of any one of claims 1 to 10 comprising
a) an analyzing unit comprising a detection agent which specifically binds to proSP-B adapted for determining the amount of proSP-B in a sample of a subject suffering from asthma; and
b) an evaluation unit for comparing the determined amount with a reference whereby it can be diagnosed whether a subject exhibits distal bronchoalveolar damage, said unit comprising a database with reference values as defined in claim 2 or 4 and a computer-implemented algorithm for carrying out a comparison as defined in claim 3 or 5.

13. The device of claim 12, wherein said analyzing unit further comprises a detection agent which specifically binds to a natriuretic peptide and/or a cardiac troponin in a sample of a subject suffering from asthma

14. A kit adapted for carrying out the method of any one of claims 1 to 10 comprising a detection agent for the biomarker proSP-B, at least one standard for a reference and instructions for carrying out the said method.

15. The kit of claim 14, wherein said kit further comprises a detection agent for a natriuretic peptide and/or a cardiac troponin.
